# EUROPEAN PATENT SPECIFICATION

(11) **EP 2 820 123 B1**
(45) Date of publication and mention of the grant of the patent: **11.10.2017**
(21) Application number: 13754938.2
(22) Date of filing: 28.02.2013
(51) Int. Cl.: C12N 5/0735, C12N 5/071, C12N 5/02

(54) **METHOD FOR GUIDING THE DERIVATION OF ENDOTHELIAL CELLS FROM HUMAN PLURIPOTENT STEM CELLS EMPLOYING TWO-DIMENSIONAL, FEEDER-FREE DIFFERENTIATION**
VERFAHREN ZUR STEUERUNG DER GEWINNUNG VON ENDOTHELZELLEN AUS MENSCHLICHEN PLURIPOTENTEN STAMMZELLEN MIT EINER ZWEIDIMENSIONALEN FEEDER-FREIEN DIFFERENZIERUNG
PROCÉDÉ POUR GUIDER LA DÉRIVATION DE CELLULES ENDOTHÉLIALES PROVENANT DE CELLULES SOUCHES PLURIPOTENTES HUMAINES À L'AIDE D'UNE DIFFÉRENCIATION BIDIMENSIONNELLE SANS CELLULES NOURRICIÈRES

(30) Priority: 29.02.2012 US 201261604884 P
(43) Date of publication of application: 07.01.2015
(73) Proprietor: The Johns Hopkins University, Baltimore, MD 21218 (US)
(72) Inventor: GERECHT, Sharon, Baltimore, MD 21212 (US); KUSUMA, Sravanti, Baltimore, MD 21210 (US)
(74) Representative: O'Brien, Lisa
(86) International application number: PCT/US2013/028341
(87) International publication number: WO 2013/130820

(56) References cited:
- EP-A1- 2 277 993
- WO-A1-03/010303
- WO-A1-2010/099539
- WO-A2-2011/090684
- WO-A2-2011/106681
- WO-A2-2012/006440
- US-A1- 2010 216 181
- US-A1- 2010 279 403
- US-A1- 2011 305 672
- US-A1- 2012 015 395
- DONNY HANJAYA-PUTRA ET AL: "Vascular endothelial growth factor and substrate mechanics regulate in vitro tubulogenesis of endothelial progenitor cells", JOURNAL OF CELLULAR AND MOLECULAR MEDICINE, vol. 14, no. 10, 1 October 2010 (2010-10-01), pages 2436-2447, XP055200855, ISSN: 1582-1838, DOI: 10.1111/j.1582-4934.2009.00981.x
- LINO S FERREIRA ET AL: "Vascular Progenitor Cells Isolated From Human Embryonic Stem Cells Give Rise to Endothelial and Smooth Muscle-Like Cells and Form Vascular Networks In Vivo", CIRCULATION RESEARCH, GRUNE AND STRATTON, BALTIMORE, US, vol. 101, no. 3, 14 June 2007 (2007-06-14) , pages 286-294, XP008157308, ISSN: 0009-7330, DOI: 10.1161/CIRCRESAHA.107.150201
- SEYED MAHMOUD HASHEMI ET AL: "The promotion of stemness and pluripotency following feeder-free culture of embryonic stem cells on collagen-grafted 3-dimensional nanofibrous scaffold", BIOMATERIALS, ELSEVIER SCIENCE PUBLISHERS BV., BARKING, GB, vol. 32, no. 30, 20 June 2011 (2011-06-20) , pages 7363-7374, XP028261565, ISSN: 0142-9612, DOI: 10.1016/J.BIOMATERIALS.2011.06.048 [retrieved on 2011-06-25]
- ELAINE VO ET AL: "Smooth-Muscle-Like Cells Derived from Human Embryonic Stem Cells Support and Augment Cord-Like Structures In Vitro", STEM CELL REVIEWS AND REPORTS, vol. 6, no. 2, 28 April 2010 (2010-04-28), pages 237-247, XP055074229, ISSN: 1550-8943, DOI: 10.1007/s12015-010-9144-3
- NOURSE, MARILYN B. ET AL.: 'VEGF induces differentiation of functional endotheliun from human embryonic stem cells' CELL BIOLOGY/SIGNALING vol. 30, 29 October 2009, pages 80 - 89, XP055163817

## Description

### Background of the Invention

### Area of the Art

The present invention is in the area of pluripotent stem cells and more particularly deals with a method to differentiate endothelial cells from stem cells.

### Description of the Background Art

Recreating functional vasculature is a pivotal step in the development of novel therapies in the field of regenerative medicine by providing innovative treatment options for patients suffering from vascular disorders through generating functional and transplantable tissues that have been engineered *in vitro.* The vascularization of tissue constructs remains a major challenge in regenerative medicine. Without its own blood supply, an engineered construct relies mainly on diffusional oxygen supply, which can only support a thin layer of viable tissue. Therefore, vascularization of a tissue construct is crucial for its successful implantation, survival, and integration with the host tissue. The formation of mature and functional vascular networks requires interaction between endothelial cells (ECs) and vascular smooth muscle cells (v-SMCs). During early vascular development, ECs line the vessel wall and organize into an immature vasculature. To further stabilize these nascent vessels, ECs secrete platelet-derived-growth-factors (PDGF) to induce the differentiation of specialized mesenchymal stem cells (MSCs) into pericytes in capillaries or SMCs in larger vessels. At this later stage, transforming growth factor-beta 1 (TGF-β1) regulates vessel maturation by inducing v-SMC differentiation and the generation of extracellular matrix (ECM) molecules, such as collagen, fibronectin, and Laminin. This process of vascular morphogenesis involving ECs interacting with both the ECM and v-SMCs has been widely studied *in vitro* using Matrigel assays. When grown on Matrigel, a basement membrane matrix enriched with laminin, ECs and v-SMCs interact to form capillary-like structures (CLSs) that resemble tube formation *in vivo.*

ECs comprise the inner lining of blood vessels and must work dynamically with blood flowing within and with SMCs, which surround and provide support to the endothelial lining of the vessel. Obtaining a well-defined and homogenous population of ECs remains a major roadblock toward the goal of vascular reconstruction. The self-renewal capability and pluripotency of human pluripotent stem cells (hPSCs) - *i.e.,* human embryonic stem cells (hESCs) and human induced PSCs (hiPSCs) - *in vitro* make them attractive for tissue engineering and vascular regenerative applications. Thus, controlled and robust differentiation of hPSCs toward vascular lineages is critical for the advancement and future of patient-specific vascular therapeutics.

Both hESCs and hiPSCs have the ability to differentiate into ECs (1, 2). Many methods to induce vascular differentiation have relied on an embryoid body (EB) intermediate, which entails spontaneous differentiation to an amalgamated cell mass in suspension and subsequent isolation of cells from the EB based on specific early endothelial markers (3, 4). Other methods incorporate of inducing vascular differentiation include co-culture with mouse stromal cells, (5, 6) which is not conducive to clinical translation. By avoiding an EB intermediate and dependence on a mouse feeder layer, our previously established method guides hPSCs differentiation toward vascular lineages in an adherent culture (7).

The current invention solves these shortcomings by deriving a homogenous population of ECs from hPSCs in a controllable and clinically relevant manner. We exploit the advantages of the adherent culture method to generate a highly homogenous EC population from hPSCs by using defined chemical compositions.

### Description of the Figures

FIGURE 1 is a schematic diagram comparing differentiation methods;
FIGURE 2 shows a number of graphs showing the determination of marker expression profile of derived cells; Fig 2A shows the analysis of VEcad, CD31, KDR, Tra-1-60 protein expression after 12 days in culture in media supplemented with 50 ng/ml VEGF, 50 ng/ml VEGF + SB431542, or 1 ng/ml VEGF + SB431542; Fig. 2B shows the expression of SMC markers over 12 days of culture; Fig. 2C shows representative flow cytometry plots of co-expression of VEcad with EC markers, CD31, CD105, and CD146, and pericyte marker, CD73;
FIGURE 3 shows immunofluorescence analysis of derived ECs examined for (A) VEcad (red), vWF (green); (B) lectin (red), CD31 (green); (C) AcLDL (red) uptake and (D) tube formation on Matrigel; nuclei in blue in (A), (B) and (C);
FIGURE 4 shows I functionality of hiPSC-derived ECs seven days after subcutaneous implantation of derived ECs (PKH26); mice were tail injected with mouse FITC-lectin (green; host vessels);explants were analyzed by confocal microscopy; nuclei in blue with purple arrows indicate chimeric vessels; white arrows mark human vessels.

### Detailed Description of the Invention

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide a method for causing pluripotent stem cells to differentiate into effective ECs.

The present inventors describe a step-wise protocol for differentiating mammalian, including human, pluripotent stem cells (PSCs) into ECs *in vitro.* The PSCs can be derived from any suitable source. For example, they can be embryonic stem cells (ESCs) or induced pluripotent stem cells (abbreviated iPS cells or iPSCs). The method, which is simple, efficient and reliable, allows for the efficient derivation of concentrated, purified, ECs. The derived ECs highly express specific endothelial cell markers. In the presence of extracellular matrix (Matrigel) the ECs mature to form linear and tubular structures. When transplanted into mammals these structures mature into actual vessels which can serve as a ready source for therapeutic vascular tissue engineering.

As used herein, the singular forms "a," "an" and "the" include plural referents unless the context clearly dictates otherwise.

Throughout this application, the term "about" is used to mean plus or minus 10% of the value. For example, about 2x10⁴ cells includes 1.8x10⁴ - 2.2 x104 cells. Ranges as used herein include the endpoints of the range.

"Pluripotent" cells, as used herein, refers to stem cells that have the potential to differentiate into any of the three germ layers: endoderm (interior stomach lining, gastrointestinal tract, the lungs), mesoderm (muscle, bone, blood, urogenital), or ectoderm (epidermal tissues and nervous system). Pluripotent stem cells can give rise to any fetal or adult cell type.

Induced pluripotent cells (commonly abbreviated as iPS cells or iPSCs) are a type of pluripotent stem cell that is artificially derived from a non-pluripotent cell, such as an adult somatic cell, by forced expression of certain genes. Methods for generating iPS cells are conventional and well-known to those of skill in the art.

Embryonic stem cells (ESCs) are described as "undifferentiated" when a substantial portion of stem cells and their derivatives in the population display morphological characteristics of undifferentiated cells, clearly distinguishing them from differentiated cells of embryonic or adult origin. Undifferentiated ES cells are easily recognized by those skilled in the art, and typically appear in a microscopic view as cells with high nuclear/cytoplasm ratios and prominent nucleoli. Similarly, undifferentiated cells can be distinguished from differentiated cells by the absence of lineage specific markers such as vascular endothelial growth factor receptor 2 (VEGFR2), vascular endothelial cadherin (VE-cad) or platelet-endothelial cell adhesion molecule-1 (PECAM-1). Often, hESCs are cultured with mouse embryonic fibroblasts (MEFs), a layer of feeder cells that nurture the hESCs and keep them in undifferentiated state.

Much of the discussion in the present application is directed to iPSCs. However, other forms of PSCs, such as ESCs, are included. In a method of the invention, in the first culture step, individual undifferentiated ES cells are cultured in a manner suitable for inducing differentiation into vasculogenic progenitor cells.

Before being plated and cultured, the PSCs, which often have been grown on a feeder layer, are treated with a suitable reagent (e.g., digested with trypsin, such as TrypLE, or treated with EDTA) to detach them from the culture plate, and are treated further to generate a single-cell suspension of cells that are smaller than about 50 µm (e.g., about 40 µm or smaller). The sizing step not only sorts the cells into cells of a desired size, but also separates them from undesirable, larger cells, such as feeder layer cells (e.g., MEFs) or EPC that may be present in the culture. Sizing methods such as filtration can also help to break up cells that have adhered to one another, e.g., in ESC colonies.

Differentiation of individual undifferentiated PSCs can be affected by culturing such cells on plates coated with an adhesive substrate such as type IV collagen, laminin or gelatin to prevent aggregation of the ES cells; seeding the cells at a low plating density (at a seeding concentration of about 5 x 10⁴ cells/ cm²- about 1 x 10⁵ cells/ cm², for example about 5 x 10⁴ cells/cm² - about 7 x 10⁴ cells/cm², or about 5 x 10⁴ cells/cm²); and providing differentiation medium that contains no growth factors. In one embodiment, individual undifferentiated ES cells are grown on type IV collagen-coated plates (available from, for example, Cell Cultureware, BD-Falcon, Boston, Mass.).

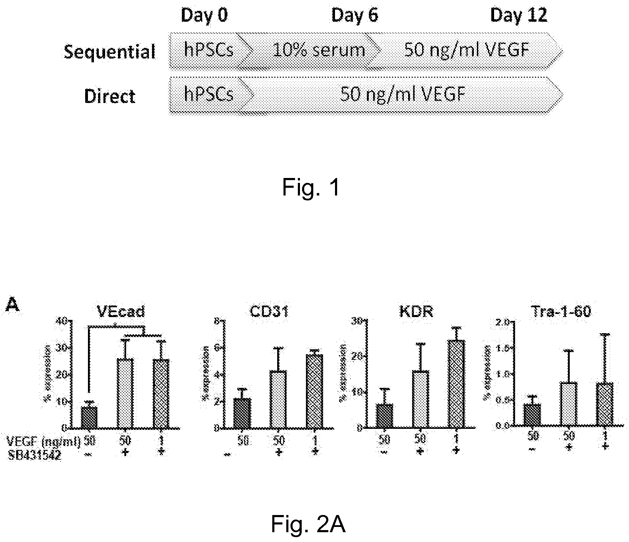

In the inventive method, hPSCs were cultured at a concentration of 1.25x10⁴ per cm² on collagen IV plates for 12 days. In the "direct" method, the cells were treated with VEGF for the entire time. With the "sequential" method, the cells were supplemented with 10% serum and were not treated with VEGF until day 6. See Fig. 1 for a comparison of the protocols.

In our sequential method, hPSCs were cultured on collagen IV plates in media supplemented with 10% serum. After 6 days in culture, cells were strained and re-cultured in media supplemented with 50 ng/ml vascular endothelial growth factor (VEGF). Cells which were differentiated via direct means were cultured in the VEGF-supplemented media for all 12 days. EC markers were examined via immunofluorescence microscopy and flow cytometry. In a reference example we tested hESC line, H9, as well as hiPSC line, MR31, which was derived from normal, fetal fibroblasts using three factors, Oct-4, Sox2, Klf4 (8, 9).

MR31 cells differentiated via the sequential scheme yielded a greater percentage of vascular endothelial cadherin positive (VEcad+) cells (∼5%) compared to the direct method (∼2%). Expression of von Willebrand factor (vWF), which is a large glycoprotein characteristic of ECs, was observed in its characteristic speckled appearance in the differentiated cells. Using quantitative image analysis, we determine that the vWF expression was significantly up-regulated via the sequential differentiation method (∼5 fold). Moreover, culture on Matrigel (BD Biosciences) revealed that cells derived via the sequential method form more homogeneous tube-like branching structures, whereas the direct differentiation method primarily yielded cells that cluster together. Similar results were obtained when the comparative analyses were performed on differentiating H9 cells. Collectively, these results indicate a sequential differentiation scheme, compared to a direct one, is more conducive to endothelial differentiation.

However, because sequentially-derived cells were only ∼5% VEcad+, we sought to improve upon the differentiation efficiency via biochemical means. To this end, we studied the addition of a TGFβ inhibitor, SB431542 (10) as well as angiogenic growth factors, bone morphogenetic protein-4 (BMP4) and Indian Hedgehog (Ihh). For our TGFβ inhibitor studies, we examined the effects of SB431542 in the second half of differentiation plus high (50ng/ml) or low (1ng/ml) VEGF. When we added SB431542 to the sequential differentiation scheme, we observed a dramatic increase in expression of mature EC marker, VEcad (Fig. 2A). Remarkably, VEcad expression increased regardless of high or low VEGF concentrations. Expression of CD31 and KDR were also increased in the presence of SB431542 (Fig. 2A). Cells expressed approximately 1% Tra-1-60 indicating they were fully differentiated. However, BMP4 or Ihh were not able to augment endothelial differentiation capacity above levels seen with SB431542 supplementation.

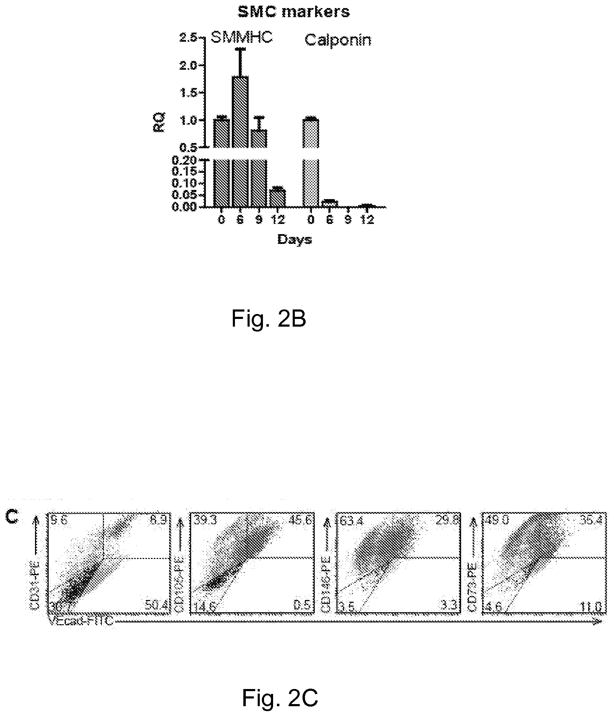

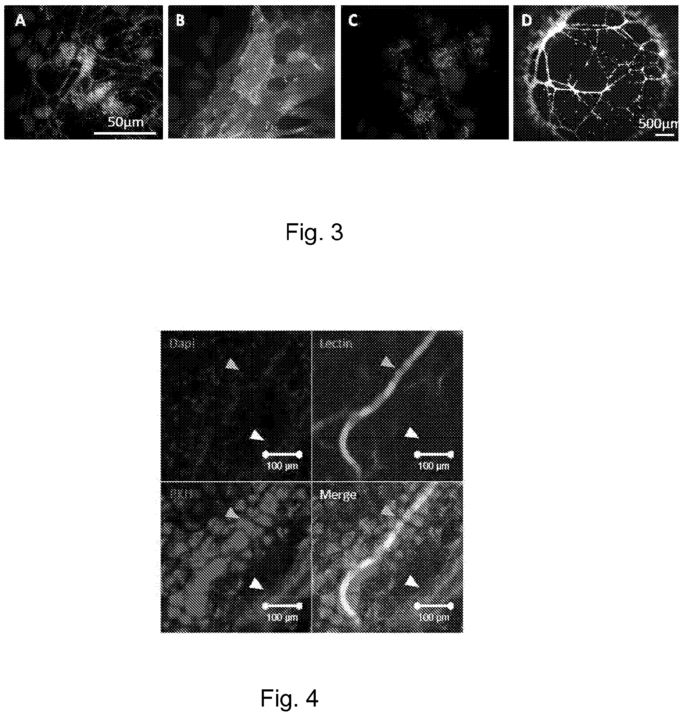

We were interested in determining whether SMCs may be present in the derived population. Quantitative RT-PCR analysis revealed that SMC markers - smooth muscle myosin heavy chain and calponin - decreased to levels less than 0.05 (relative to day 0 cells) after 12 days, suggesting little to no SMC presence in our cultures (Fig 2B).To establish a complete marker profile of the derived cells, we examined co-expression of VEcad with EC markers - CD31, CD105 and CD146 - and with pericyte markers - CD73 and NG2. We observed that a subset of our cells were CD31+VEcad+ (∼8%; Fig 2C). Our derived cells were enriched in CD105, CD146, and CD73 (Fig 2C), but NG2 was not detected (data not shown). Further inquiry into the kinetics of marker expression along the course of differentiation demonstrated that expression of EC markers, VEcad, CD31, and KDR increased from day 6 to 12. Sub-culture of day 12 cells for an additional 6 days yielded further enrichment of VEcad, vWF, CD31, and lectin which were all properly localized (Fig. 3A, 3B).

After optimizing our 2D differentiation scheme, we investigated whether the derived ECs exhibited characteristic endothelial functionalities. Mature ECs are known for their ability to uptake acetylated low density lipoprotein (AcLDL) as part of LDL metabolism in the body and their capacity to commence vasculogenesis upon culture on Matrigel. Upon incubation with labeled AcLDL, a fraction of the derived cells exhibited internalized AcLDL (Fig. 3C). When our derived ECs were cultured on Matrigel, we observed cord-like structures after 24 hours (Fig. 3D).

*In vivo* functionality is crucial to the success of derived ECs toward tissue engineering purposes. To ensure our derived ECs were able to survive implantation, form vascular networks, integrate with the host vasculature, and establish blood flow, the cells were encapsulated in Matrigel and subcutaneously implanted in nude mice. Derived ECs were labeled with PKH-26 (11) for ease of visualization in explanted tissue. To visualize angiogenesis in the implants prior to sample removal, mice were injected intravenously with anti-mouse FITC-lectin to enable visualization of mouse blood vessels within red-labeled (PKH) human vasculature using fluorescent microscopy. After 1 week, implants were harvested and imaged via confocal microscopy. Fig. 4 shows the results; the DAPI image shows the cell nuclei; the lectin image shows the FITC labeled mouse tissue while the PKH image shows the human tissue. The "Merge" image shows the other 3 images overlapped. We found that our derived ECs were not only able to incorporate into the mouse vasculature forming chimeric vessels (Fig. 4, purple [upper] arrows), but cells also formed their own human vessels, devoid of mouse cells (Fig. 4, white [lower] arrows).

Collectively, these findings reveal that hPSCs treated according to our method are able to differentiate into ECs which exhibit the appropriate marker expression profiles and are functional both *in vitro* and *in vivo.*

Thus, we have developed chemically defined conditions for the controlled differentiation and robust derivation of functional ECs from both embryonic and induced pluripotent stem cells. The derived cells exhibit important functional characteristics of ECs such as uptake of AcLDL, cord formation on Matrigel, and incorporation in vivo. Because our cells are derived via controlled conditions in a 2D manner, these studies establish an efficient and clinically relevant methodology for deriving functional ECs. This invention will have considerable clinical impact with respect to improved vascular therapeutics and regenerative medicine.

The following claims are thus to be understood to include what is specifically illustrated and described above, what is conceptually equivalent, what can be obviously substituted and also what incorporates the essential idea of the invention. The illustrated embodiment has been set forth only for the purposes of example and that should not be taken as limiting the invention. Therefore, it is to be understood that, within the scope of the appended claims, the invention may be practiced other than as specifically described herein.

### References :

1. S.I. Nishikawa, et al., Development, 125, 1747-1757 (1998).
2. S. Levenberg, et al., Blood, 110, 806-814 (2007).
3. S. Levenberg, et al., PNAS, 99, 4391-4396 (2002).
4. L.S. Ferreira, et al., Circulation Research, 101, 286-294 (2007).
5. M.A. Vodyanik and I.I. Slukvin, Current protocols in cell biology, Chapter 23 (2007).
6. K.L. Hill, et al., Exp Hematol, 38, 246-257 e241.
7. S. Gerecht-Nir, et al., Laboratory Investigation, 83, 1811-1820 (2003).
8. P. Mali, et al., Stem Cells, 28, 713-720 (2010).
9. A. Swistowski, et al., Stem Cells, 28, 1893-1904 (2010).
10. D. James, et al., Nature Biotechnology, 28, 161-166 (2010).
11. J.W. Ford et al., J. Surg. Res., 62, 23-28 (1996).
The application discloses:
1. A method for differentiating mammalian pluripotent stem cells (PSCs) into endothelial cells (ECs) in vitro, comprising the steps of:
   plating a single-cell suspension of PSCs onto a suitable surface;
   culturing the cells in the presence of VEGF; and
   harvesting the cultured cells whereby ECs are produced.
2. The method for differentiating PSCs of clause 1, wherein the VEGF ranged from 1 to 50 ng/ml.
3. The method for differentiating PSCs of clause 1, wherein the suitable surface is type IV collagen.
4. The method for differentiating PSCs of clause 1 further comprising a step of culturing in the absence of VEGF prior to the step of culturing in the presence of VEGF.
5. The method for differentiating PSCs of clause 4, wherein the media used for culturing are supplemented with serum.
6. The method for differentiating PSCs of clause 5, wherein the media are supplemented with 10% serum.
7. The method for differentiating PSCs of clause 4, wherein the step of culturing in the presence of VEGF also includes culturing in the presence of a transforming growth factor-beta (TGF-β) inhibitor.
8. The method for differentiating PSCs of clause 7, wherein the TGF-β inhibitor is SB431542.
9. The method for differentiating PSCs of clause 4, wherein the step of culturing in the absence of VEGF lasts for several days prior to the step of culturing in the presence of VEGF.
10. The method for differentiating PSCs of clause 4, wherein the cells are harvested from culturing in the absence of VEGF and then cultured in the presence of VEGF.
11 . A method for differentiating mammalian pluripotent stem cells (PSCs) into endothelial cells (ECs) in vitro, comprising the steps of:
   plating a single-cell suspension of PSCs onto a surface od type IV collagen;
   culturing the cells without VEGF
   culturing the cells in the presence of VEGF; and
   harvesting the cultured cells whereby ECs are produced.
12. The method for differentiating PSCs of clause 11, wherein the VEGF ranged from 1 to 50 ng/ml.
13. The method for differentiating PSCs of clause 11, wherein the media used for culturing are supplemented with serum.
14. The method for differentiating PSCs of clause 13, wherein the media are supplemented with 10% serum.
15. The method for differentiating PSCs of clause 11, wherein the step of culturing in the presence of VEGF also includes culturing in the presence of a transforming growth factor-beta (TGF-β) inhibitor.
16. The method for differentiating PSCs of clause 15, wherein the TGF-β inhibitor is SB431542.
17. The method for differentiating PSCs of clause 11, wherein the step of culturing without VEGF lasts for several days prior to the step of culturing in the presence of VEGF.
18. The method for differentiating PSCs of clause 11, wherein the cells are harvested from culturing without VEGF and then cultured in the presence of VEGF.

## Claims

1. A method for differentiating mammalian pluripotent stem cells (PSCs) into endothelial cells (ECs) *in vitro,* comprising the steps of:
plating a single-cell suspension of PSCs at a seeding concentration of 1.25x10⁴ cells/cm² - 1x10⁵ cells/cm² onto an adhesive substrate;
culturing the cells in the presence of VEGF for 1 to 12 days, wherein the VEGF ranged from 1 to 50 ng/ml; and
harvesting the cultured cells whereby ECs are produced.

2. The method of claim 1, wherein the adhesive substrate is type IV collagen, and wherein the method further comprises culturing the cells in the absence of VEGF for 1 to 6 days prior to the step of culturing the cells in the presence of VEGF.

3. The method for differentiating PSCs of claim 1, wherein the adhesive substrate is type IV collagen.

4. The method for differentiating PSCs of claim 1 further comprising a step of culturing in the absence of VEGF for 1 to 6 days prior to the step of culturing in the presence of VEGF.

5. The method for differentiating PSCs of claim 2 or claim 4, wherein the media used for culturing are supplemented with serum.

6. The method for differentiating PSCs of claim 5, wherein the media are supplemented with 10% serum.

7. The method for differentiating PSCs of claim 2 or claim 4, wherein the step of culturing in the presence of VEGF also includes culturing in the presence of a transforming growth factor-beta (TGF-β) inhibitor.

8. The method for differentiating PSCs of claim 7, wherein the TGF-β inhibitor is SB431542.

9. The method for differentiating PSCs of claim 4, wherein the cells are harvested from culturing in the absence of VEGF and then cultured in the presence of VEGF.

10. The method for differentiating PSCs of claim 2, wherein the cells are harvested from culturing without VEGF and then cultured in the presence of VEGF.

11. The method of claim 2 or 4, wherein the two-dimensional single-cell suspension of PSCs consists of PSCs smaller than 50 µm.

12. The method of claim 11, wherein the PSCs smaller than 50 µm are generated by a method comprising a filtration step.

## Patentansprüche

1. Ein Verfahren zum *In*-*vitro*-Differenzieren pluripotenter Stammzellen (PSC) von Säugetieren zu Endothelzellen (EC), beinhaltend die folgenden Schritte:
Ausplattieren einer Einzelzellsuspension von PSC mit einer Impfkonzentration von 1,25 x 10⁴ Zellen/cm² bis 1 x 10⁵ Zellen/cm² auf ein Haftsubstrat;
Kultivieren der Zellen in Gegenwart von VEGF für 1 bis 12 Tage, wobei der VEGF im Bereich von 1 bis 50 ng/ml liegt; und
Entnehmen der kultivierten Zellen, wodurch EC produziert werden.

2. Verfahren gemäß Anspruch 1, wobei das Haftsubstrat ein Typ-IV-Kollagen ist und wobei das Verfahren ferner das Kultivieren der Zellen in Abwesenheit von VEGF für 1 bis 6 Tage vor dem Schritt des Kultivierens der Zellen in Gegenwart von VEGF beinhaltet.

3. Verfahren zum Differenzieren von PSC gemäß Anspruch 1, wobei das Haftsubstrat Typ-IV-Kollagen ist.

4. Verfahren zum Differenzieren von PSC gemäß Anspruch 1, das ferner einen Schritt des Kultivierens in Abwesenheit von VEGF für 1 bis 6 Tage vor dem Schritt des Kultivierens in Gegenwart von VEGF beinhaltet.

5. Verfahren zum Differenzieren von PSC gemäß Anspruch 2 oder Anspruch 4, wobei die zum Kultivieren verwendeten Medien mit Serum ergänzt sind.

6. Verfahren zum Differenzieren von PSC gemäß Anspruch 5, wobei die Medien mit 10 % Serum ergänzt sind.

7. Verfahren zum Differenzieren von PSC gemäß Anspruch 2 oder Anspruch 4, wobei der Schritt des Kultivierens in Gegenwart von VEGF auch das Kultivieren in Gegenwart eines Inhibitors des transformierenden Wachstumsfaktors beta (TGF-β) umfasst.

8. Verfahren zum Differenzieren von PSC gemäß Anspruch 7, wobei der TGF-β-Inhibitor SB431542 ist.

9. Verfahren zum Differenzieren von PSC gemäß Anspruch 4, wobei die Zellen aus der Kultivierung in Abwesenheit von VEGF entnommen und dann in Gegenwart von VEGF kultiviert werden.

10. Verfahren zum Differenzieren von PSC gemäß Anspruch 2, wobei die Zellen aus der Kultivierung ohne VEGF entnommen und dann in Gegenwart von VEGF kultiviert werden.

11. Verfahren gemäß Anspruch 2 oder 4, wobei die zweidimensionale Einzelzellsuspension von PSC aus PSC besteht, die kleiner als 50 µm sind.

12. Verfahren gemäß Anspruch 11, wobei die PSC, die kleiner als 50 µm sind, durch ein Verfahren erzeugt werden, das einen Filtrationsschritt beinhaltet.

## Revendications

1. Une méthode pour la différenciation de cellules souches pluripotentes (CSP) de mammifères en cellules endothéliales (CE) *in vitro,* comprenant les étapes de :
mise en culture sur plaque d'une suspension de cellules uniques de CSP à une densité d'ensemencement de 1,25 x 10⁴ cellules/cm² à 1 x 10⁵ cellules/cm² sur un substrat adhésif ;
culture des cellules en la présence de VEGF pendant 1 à 12 jours, où le VEGF variait de 1 à 50 ng/ml ; et
récolte des cellules cultivées moyennant quoi les CE sont produites.

2. La méthode de la revendication 1, où le substrat adhésif est du collagène de type IV, et où la méthode comprend en outre la culture des cellules en l'absence de VEGF pendant 1 à 6 jours avant l'étape de culture des cellules en la présence de VEGF.

3. La méthode pour la différentiation de CSP de la revendication 1, où le substrat adhésif est du collagène de type IV.

4. La méthode pour la différentiation de CSP de la revendication 1 comprenant en outre une étape de culture en l'absence de VEGF pendant 1 à 6 jours avant l'étape de culture en la présence de VEGF.

5. La méthode pour la différentiation de CSP de la revendication 2 ou de la revendication 4, où les milieux utilisés pour la culture sont complémentés avec du sérum.

6. La méthode pour la différentiation de CSP de la revendication 5, où les milieux sont complémentés avec 10 % de sérum.

7. La méthode pour la différentiation de CSP de la revendication 2 ou de la revendication 4, où l'étape de culture en la présence de VEGF inclut aussi la culture en la présence d'un inhibiteur du facteur de croissance transformant bêta (TGF-β).

8. La méthode pour la différentiation de CSP de la revendication 7, où l'inhibiteur du TGF-β est SB431542.

9. La méthode pour la différentiation de CSP de la revendication 4, où les cellules sont récoltées de la culture en l'absence de VEGF puis cultivées en la présence de VEGF.

10. La méthode pour la différentiation de CSP de la revendication 2, où les cellules sont récoltées de la culture sans VEGF puis cultivées en la présence de VEGF.

11. La méthode de la revendication 2 ou de la revendication 4, où la suspension de cellules uniques bidimensionnelle de CSP consiste en des CSP d'une taille inférieure à 50 µm.

12. La méthode de la revendication 11, où les CSP d'une taille inférieure à 50 µm sont générées par une méthode comprenant une étape de filtration.
